# EUROPÄISCHE PATENTSCHRIFT

(11) **EP 1 636 581 B1**
(45) Veröffentlichungstag und Bekanntmachung des Hinweises auf die Patenterteilung: **10.12.2008**
(21) Anmeldenummer: 04739865.6
(22) Anmeldetag: 15.06.2004
(51) Int. Cl.: G01N 33/30

(54) **VORRICHTUNG UND VERFAHREN ZUR AUTOMATISCHEN BERWACHUNG DES GEBRAUCHSZUSTANDES EINES SCHMIERSTOFFES EINER MASCHINE ODER EINES MASCHINENTEILS**
DEVICE AND METHOD FOR AUTOMATICALLY MONITORING THE OPERATING CONDITION OF A LUBRICANT OF A MACHINE OR MACHINE PART
DISPOSITIF ET PROCEDE POUR LA SURVEILLANCE AUTOMATIQUE DE L'ETAT D'UTILISATION D'UN LUBRIFIANT D'UNE MACHINE OU D'UNE PIECE DE MACHINE

(30) Priorität: 20.06.2003 DE 10327625
(43) Veröffentlichungstag der Anmeldung: 22.03.2006
(73) Patentinhaber: Fuchs Petrolub AG, 68169 Mannheim (DE)
(72) Erfinder: DITTMANN, Brigitte, 80638 München (DE); KESSELER, Angela, 46047 Oberhausen (DE); LUTHER, Rolf, 67346 Speyer (DE); MEINDORF, Thomas, 76689 Karlsdorf (DE); PLENERT, Frank, 68623 Lampertheim-Hüttenfeld (DE); SEYFERT, Christian, 68167 Mannheim (DE); ZEIDLER, Carsten, 85354 Freising (DE)
(74) Vertreter: Reiser, Tonio Andreas
(86) Internationale Anmeldenummer: PCT/EP2004/006385
(87) Internationale Veröffentlichungsnummer: WO 2004/113689

(56) Entgegenhaltungen:
- EP-A- 0 338 744
- US-A- 4 890 478
- US-A- 6 037 592
- US-B1- 6 421 588

## Beschreibung

Die vorliegende Erfindung betrifft eine Vorrichtung zum automatischen Überwachen des Gebrauchszustandes eines Schmierstoffes einer Maschine oder eines Maschinenteils, mit einer Probenkammer, welche einen Raum zur Aufnahme einer Schmierstoffprobe, wenigstens einen Einlass und Auslass für Trägergas und einen Gasraum über der Schmierstoffprobe, in dem sich das Trägergas mit flüchtigen Bestandteilen des Schmierstoffes anreichern kann, aufweist, und mit wenigstens einem Gassensor, der ein von der Konzentration eines oder mehrerer flüchtiger Bestandteile in dem angereicherten Trägergas abhängiges Signal erzeugt. Die vorliegende Erfindung betrifft darüber hinaus ein Verfahren zum automatischen Überwachen des Gebrauchszustandes eines Schmierstoffes einer Maschine oder eines Maschinenteils.

Die Begriffe Schmierstoff und Schmierstoffprobe in der vorliegenden Anmeldung sollen sämtliche Stoffe umfassen, deren Haut- oder Nebenfunktion die Schmierung von Maschinenteilen ist. Hierunter fallen insbesondere flüssige und konsistente Schmierstoffe, wie Schmieröle und Schmierfette, Hydraulikflüssigkeiten, Metallbearbeitungsflüssigkeiten, Transformatorenöle und dergleichen. Die vorliegende Anmeldung bezieht sich auf sämtliche dieser vorgenannten Stoffe.

Die Überwachung von Schmierstoffen von Maschinen bzw. Maschinenteilen dient der Erhöhung von deren Betriebssicherheit und Anlagenverfügbarkeit sowie der Minimierung unnötiger oder vorzeitiger Schmierstoffwechsel. Derartige Überwachungen erfolgten bisher überwiegend mittels Analyse von Flüssigkeitsproben, die in diskreten Zeitabständen von spezialisierten Labors oder durch geeignete Laborgeräte vor Ort untersucht werden.

Um eine kontinuierliche Überwachung von Maschinen zu ermöglichen und die Kosten zu reduzieren, sind bereits automatische Zustandsüberwachungen beschrieben worden.

Die gängigsten zu diesem Zweck eingesetzten Sensoren sind sogenannte dekametrische Sensoren zur Messung der Dielektrizitätskonstante, der Leitfähigkeit und/oder dem dielektrischen Verlustfaktor im Öl. Derartige Sensoren werden heute in verschiedenen Varianten in großer Stückzahl zur Motorölüberwachung eingesetzt. Darüberhinaus sind Viskositätssensoren kommerziell verfügbar. Die Kombination der Messung von Dielektrizität und Viskositätsänderung ergibt sich beispielsweise aus der DE 197 06 486 A1. Bei dem darin beschriebenen Verfahren wird der Sensor mit der zu überwachenden Flüssigkeit in direkten Kontakt gebracht. Sensoren die in permanenten Kontakt mit dem zu überwachenden Stoff stehen, haben den Nachteil, dass sie besonderen Alterungs- und Verschmutzungserscheinungen ausgesetzt sind. Ein typisches Problem ist beispielsweise die Belagbildung aufgrund von klebrigen Ölalterungsprodukten.

Neben diesen Verfahren, die direkt in der zu überwachenden Flüssigkeit arbeiten, sind bereits Verfahren beschrieben worden, bei denen die Qualität von Fetten und Ölen mittels der Detektion von flüchtigen Stoffen erfolgt. Ein solches Verfahren ist in der DE 199 47 669 A1 beschrieben, bei dem die Öl- bzw. Fettqualität durch Gassensoren ermittelt wird, welche die Konzentration von schwer flüchtigen Kohlenwasserstoffen bestimmen. Zusätzlich ist in diesem Dokument die Messung leicht flüchtiger Kohlenwasserstoffe und die Verwendung des Verhältnisses leicht zu schwer flüchtigen Kohlenwasserstoffen als Maß für die Fett- bzw. Ölqualität beschrieben. Als Korrekturfaktoren werden Öltemperaturen und Ölfeuchte genannt, die zusätzlich bestimmt werden müssen.

Die US 6,421,588 B1 beschreibt ein Verfahren, bei dem ein Sensorarray die Gaszusammensetzung über dem Ölsumpf bzw. einem Ölreservoir eines Schmierölumlaufsystems während des Abkühlens bestimmt. Aus den gemessenen Temperaturen des Sensors und des Öls sowie aus der temperaturabhängigen Gaszusammensetzung soll auf den Zustand des Schmieröls geschlossen werden.

Nachteilig bei den aus der DE 199 47 669 sowie US 6,421,588 bekannten Verfahren ist, dass die Messergebnisse stark schwanken und von den Randbedingungen am Ort der Messung abhängig sind.

Das Dokument EP-A 0 338 744 beschreibt eine Vorrichtung und ein Verfahren zum Messen von gelöstem Gas in einem Öl, wobei ein Luftblasen-Generator vorgesehen ist. Die Luft wird als Trägergas benutzt und durch die Ölprobe in eine Probenkammer geleitet. Diese Vorrichtung ist jedoch nicht zur automatischen Überwachung des Gebrauchszustandes eines Schmierstoffes einer Maschine oder eines Maschinenteils geeignet. Zudem hat die vorbekannte Vorrichtung keine Mittel zur Einstellung der physikalischen Bedingungen in der Probenkammer.

Das Dokument US-6 037 592 beschreibt eine Vorrichtung und ein Verfahren zum Überwachen einer Gaskonzentration in einer Flüssigkeit ohne Trägergas mit Mitteln zur Einstellung der Temperatur der Flüssigkeit in dem System.

Der vorliegenden Erfindung liegt daher die Aufgabe zugrunde, eine Vorrichtung und ein Verfahren zum automatischen Überwachen des Gebrauchszustandes eines Schmierstoffes einer Maschine oder eines Maschinenteils anzugeben, das eine hohe Zuverlässigkeit aufweist.

Diese Aufgabe wird bei einer Vorrichtung der eingangs genannten Art dadurch gelöst, dass Mittel zur Einstellung der physikalischen Bedingungen in der Probenkammer vorgesehen sind.

Die erfindungsgemäße Vorrichtung erlaubt die laufende und automatische Überwachung von Schmierstoffen, insbesondere von Schmierölen und Schmierfetten sowie Hydraulik- und Metallbearbeitungsflüssigkeiten, Transformatorölen und verwandten Flüssigkeiten in der Anlage ohne manuelle Probenentnahme. Die Bestimmung und Beurteilung des Gebrauchszustandes der zu überwachenden Stoffe erfolgt insbesondere mit der relativen und/oder absoluten Konzentrationsbestimmung charakteristischer flüchtiger Substanzen und/oder Substanzgruppen dieser Stoffe. Die erfindungsgemäße Vorrichtung gewährleistet die optimale Auswahl und Einstellung aller Randbedingungen, insbesondere von Temperatur und Druck in der Probenkammer, welche den qualitativen und quantitativen Nachweis von flüchtigen Stoffbestandteilen mit den Sensoren beeinflussen können. Damit können Messergebnisse erhalten werden, die von Bedingungen wie der Temperatur der Maschine etc. nicht oder nur unwesentlich beeinflusst werden.

Eine weitere Verbesserung der Messwerte wird dadurch erreicht, dass der wenigstens eine Gassensor in einer Sensorkammer angeordnet ist und dass der Gasraum der Probenkammer mit der Sensorkammer durch eine Zufuhrleitung verbunden ist.

Die Messergebnisse können dadurch weiter verbessert werden, dass Mittel zur Einstellung der physikalischen Bedingungen in der Sensorkammer vorgesehen sind. Auf diese Weise können z.B. Temperatur und/oder Druck nicht nur in der Probenkammer, sondern auch in der Sensorkammer in einem weiten Bereich geregelt werden.

Vorteilhafterweise ist wenigstens ein Trägergasventil in der Zufuhrleitung zwischen der Probenkammer und der Sensorkammer vorgesehen.

Gemäß einer vorteilhaften Ausgestaltung dieses Erfindungsgedankens ist vorgesehen, dass durch das wenigstens eine Trägergasventil in der Zufuhrleitung zwischen Probenkammer und Sensorkammer die Zufuhr von Trägergas aus der Probenkammer in die Sensorkammer ausgelöst und/oder unterbunden werden kann. Hierdurch kann bei geschlossenem Trägergasventil eine Verweilzeit des Trägergases in dem Gasraum der Probenkammer erreicht und die Einstellung des Adsorptions-Desorptionsgleichgewichts zwischen flüssiger Phase, Gasphase und den Wänden der Probenkammer abgewartet werden. Dies ist wichtig, um reproduzierbare Ergebnisse und eine hohe Messgenauigkeit zu erreichen. Nach Ablauf der gewünschten Verweilzeit, in der sich das Trägergas mit den flüchtigen Bestandteilen des zu untersuchenden Mediums anreichert, kann das Trägergasventil geöffnet und auf diese Weise der Weg für das angereicherte Trägergas aus der Probenkammer in die Sensorkammer frei gemacht werden.

Gemäß einer vorteilhaften Weiterbildung der Erfindung ist die Probenkammer und/oder die Sensorkammer mit einer über Thermostat gesteuerten Heizung und/oder Kühlung versehen.

Gemäß einer vorteilhaften Ausgestaltung der Erfindung sind Mittel zur Zufuhr einer Schmierstoffprobe aus dem Schmiersystem einer Maschine oder eines Maschinenteils, insbesondere aus dessen Schmierstoffkreislauf, in die Probenkammer vorgesehen. Die Mittel zur Zufuhr einer Schmierstoffprobe können insbesondere als ein automatisches System ausgebildet sein, das die Probenkammer z.B. in regelmäßigen Abständen mit einer gleichbleibenden Menge des zu untersuchenden Stoffes füllt. Hierdurch kann eine weitere Verbesserung der Messergebnisse erreicht werden, da in der Probenkammer stets die gleiche Menge Schmierstoff und Trägergas enthalten ist. Der Transport von flüssigen zu untersuchenden Stoffen in die Probenkammer und aus der Probenkammer zurück in die Anlage kann durch kleine Pumpen, den Eigendruck der Anlage, z.B. bei der Überwachung einer Hydraulikflüssigkeit, Gravitation und/oder durch den Druck des Trägergases erfolgen.

Hierzu weist die Probenkammer vorteilhafter Weise wenigstens einen Einlass und wenigstens einen Auslass für Schmierstoff auf.

Gemäß einer Weiterbildung dieses Erfindungsgedankens ist vorgesehen, dass der Auslass derart ausgebildet ist, dass im wesentlichen eine vollständige Entleerung der Probenkammer erfolgen kann.

Gemäß einem weiteren vorteilhaften Aspekt der vorliegenden Erfindung sind Mittel zum Einbringen von Trägergas in die Probenkammer vorgesehen. Als Trägergas kann insbesondere Umgebungsluft, Pressluft oder ein Inertgas eingesetzt werden. Indem das Trägergas mittels eines Filters, z.B. eines Absorptionsfilters, von den Substanzen die nachgewiesen werden sollen und/oder von Wasserdampf befreit wird, kann die Messgenauigkeit weiter verbessert werden. Dabei erfolgt die Zufuhr von Trägergas zur Probenkammer vorteilhafterweise unter Überdruck.

Vorteilhafterweise ist weiterhin eine Einrichtung zum Spülen der Probenkammer und/oder der Sensorkammer mittels Trägergas vorgesehen. Hierdurch wird ermöglicht, vor oder nach der Durchführung von Messungen die Probenkammer und/oder die Sensorkammer mit den darin angeordneten Sensoren mit Trägergas zu spülen, so dass die Analyten desorbiert werden.

Eine vorteilhafte Weiterbildung dieses Erfindungsgedankens sieht vor, dass zum Spülen eine Spülleitung vorgesehen ist, welche die Zufuhr von sauberem Trägergas in die Sensorkammer ermöglicht.

Gemäß einem weiteren Aspekt der Erfindung ist eine Steuerung für das wenigstens eine Trägergasventil vorgesehen, welche intermittierend die Füllung der Sensorkammer mit angereichertem Trägergas ermöglicht.

Die vorliegende Erfindung bezieht sich darüber hinaus auf ein Verfahren zur automatischen Überwachung des Gebrauchszustandes eines Schmierstoffes einer Maschine oder eines Maschinenteils mit folgenden Verfahrensschritten:
- Einbringen einer Schmierstoffprobe aus der Maschine in eine Probenkammer und Einbringen von Trägergas in die Probenkammer,
- Einstellen der physikalischen Probenbedingungen in der Probenkammer auf einen vorbestimmten Wert,
- Anreicherung des Trägergases mit flüchtigen Bestandteilen der Schmierstoffprobe,
- Erfassung von wenigstens einer in dem angereicherten Trägergas enthaltenen charakteristischen flüchtigen Substanz und/oder Substanzgruppe aus der Schmierstoffprobe mit wenigstens einem Gassensor.

Aufgrund der vorgenannten Verfahrensschritte ist es möglich, die Messgenauigkeit-wesentlich zu erhöhen. Dies ist insbesondere darauf zurückzuführen, dass aufgrund der erfindungsgemäßen Gestaltung des Verfahrens die die Messung beeinflussenden Faktoren minimiert werden können. Damit ist es möglich unabhängig von dem Betriebszustand der zu überwachenden Maschine, beispielsweise dessen Temperatur oder dessen Schmierstofffüllstand, die Randbedingungen einzustellen, welche den qualitativen und quantitativen Nachweis von flüchtigen Stoffbestandteilen mit den Sensoren beeinflussen können. Die Anreicherungsphase des Trägergases in der Probenkammer wird unter genau definierten Randbedingungen durchgeführt werden. Insbesondere ist es sinnvoll Temperatur und/oder Druck in Probenkammer entsprechend einzustellen. Die Steuerung der Temperatur in den Kammern kann dabei durch thermostatisierte Heizungen erfolgen.

Vorteilhafterweise wird das angereicherte Trägergas nach der Anreicherung mit flüchtigen Bestandteilen über eine Zufuhrleitung in eine Sensorkammer geleitet, in der die Erfassung von wenigstens einer in dem angereicherten Trägergas enthaltenen charakteristischen flüchtigen Substanz und/oder Substanzgruppe aus der Schmierstoffprobe mit dem wenigstens einen Gassensor erfolgt. Diese Anordnung mit von der Probenkammer getrennter Sensorkammer machte eine weitere Verbesserung der Messergebnisse möglich. Hierbei ist es möglich auch in der Sensorkammer Temperatur und/oder Druck einzustellen. Alternativ können der wenigstens eine Gassensor auch in der Probenkammer angeordnet sein.

Gemäß einer vorteilhaften Ausgestaltung der Erfindung wird die Zufuhr des angereicherten Trägergases durch Öffnen eines in der Zufuhrleitung angeordneten Trägergasventils ausgelöst und durch Schließen des Trägergasventils unterbrochen.

Weiterhin kann erfindungsgemäß vorgesehen sein, dass das Trägergas in die Probenkammer unter Überdruck eingebracht wird. Auf diese Weise lässt sich auch einfach der Druck in der Probenkammer auf den gewünschten Wert einstellen.

Gemäß einer Weiterbildung dieses Erfindungsgedankens ist vorgesehen, dass das angereicherte Trägergas aus der Probenkammer aufgrund Entspannens des Überdrucks in der Probenkammer durch Öffnen des in der Zufuhrleitung zur Sensorkammer angeordneten Trägergasventils in die Sensorkammer transportiert wird.

Eine weitere Verbesserung der Messergebnisse wird dadurch erzielt, dass für aufeinanderfolgende Messungen stets die gleiche Menge Schmierstoffprobe und/oder Trägergas in die Probenkammer eingebracht wird.

Die Messgenauigkeit wird dadurch weiter verbessert, dass die Sensorkammer nach einer oder mehreren Messungen mit sauberem Trägergas gespült wird. Mit solchem Trägergas, welches wenig oder keine flüchtige Bestandteile des Schmierstoffes aufweist, können Verunreinigungen, welche sich ggf. in der Sensorkammer und an den Sensoren niedergeschlagen haben, entfernt oder zumindest reduziert werden.

Die Spülung kann besonders einfach durchgeführt werden, wenn die in der Probenkammer enthaltene Schmierstoffprobe entfernt und dann Trägergas durch die Probenkammer und ggf. nachfolgend durch die Sensorkammer geleitet wird.

Eine weitere Möglichkeit besteht darin, eine in die Sensorkammer mündende Spülleitung vorzusehen, durch welche die Spülung erfolgt.

Die Messergebnisse werden dadurch weiter verbessert, dass das Trägergas vor dem Einbringen in die Probenkammer gereinigt, insbesondere gefiltert wird. Als Filter können insbesondere Aktivkohlefilter oder Molekularsiebe eingesetzt werden, welche in dem Trägergas enthaltene Verunreinigungen, insbesondere Öl oder Wasser abscheiden.

Erfindungsgemäß werden als Trägergas vorzugsweise Umgebungsluft, Pressluft oder Inertgas eingesetzt.

Besonders gute Messergebnisse werden dann erreicht, wenn durch einen der Gassensoren polare charakteristische flüchtige Substanzen und/oder Substanzgruppen oder deren Konzentration und durch wenigstens einen weiteren Gassensor nicht alterungsspezifische, insbesondere unpolare charakteristische flüchtige Substanzen und/oder Substanzgruppen oder deren Konzentration detektiert werden. Dabei kann das Verhältnis von polaren zu nichtalterungsspezifischen flüchtigen Substanzen und/oder Substanzgruppen ausgewertet und als Maß für den Gebrauchszustand des Öls verwendet werden.

Weitere Ziele, Merkmale, Vorteile und Anwendungsmöglichkeiten der vorliegenden Erfindung ergeben sich aus der nachfolgenden Beschreibung von Ausführungsbeispielen anhand der Zeichnung. Dabei bilden alle beschriebenen und/oder bildlich dargestellten Merkmale für sich oder in beliebiger Kombination den Gegenstand der Erfindung, auch unabhängig von der Zusammenfassung in einzelnen Ansprüchen oder deren Rückbeziehung.

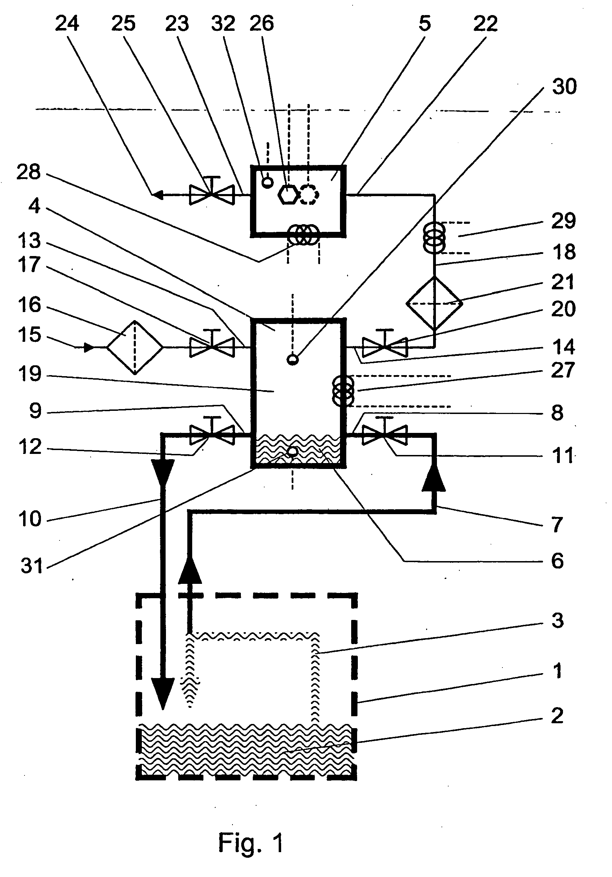

Figur 1 zeigt eine erfindungsgemäße Vorrichtung.

In Figur 1 ist eine erfindungsgemäße Vorrichtung schematisch dargestellt, welche insbesondere für flüssige Schmierstoffe wie Schmieröl geeignet ist. Vorliegend wird die Ausführungsform anhand einer technischen Anlage mit einem Schmierölkreislauf erläutert. Die zu überwachende Anlage 1 weist ein Ölreservoir 2 und eine Ölumlaufschmierung 3 auf.

Die erfindungsgemäße Vorrichtung weist eine Probenkammer 4 auf, welche einen Raum zur Aufnahme einer Schmierstoffprobe bildet. Zur Befüllung der Probenkammer 4 mit einer definierten Menge Schmierstoff sind Mittel zur Zufuhr einer Schmierstoffprobe aus dem Schmiersystem 3 der Anlage 1 vorgesehen. Bei der in Figur 1 dargestellten Ausführungsform wird hierzu ein Teilstrom des Schmierstoffkreislaufs über die Ölzuleitung 7 über einen Einlass 8 in die Probenkammer 4 geleitet. Über einen Auslass 9 und die Ölablaufleitung 10 kann die in der Probenkammer 4 enthaltene Schmierstoffprobe 6 wieder zurück in das Ölreservoir 2 gefördert werden. Mittels der in der Ölzulaufleitung 7 und in der Ölablaufleitung 10 vorgesehenen Ventile 11, 12 kann die in der Probenkammer 4 aufgenommene Flüssigkeitsmenge genau eingestellt werden.

Die Probenkammer 4 verfügt darüber hinaus über einen Trägergaseinlass 13 und einen Trägergasauslass 14. Das Trägergas in der Probenkammer 4 stammt dabei aus einer Gasversorgung 15 mit einem definierten Druck p_{G}. Das Trägergas, welches insbesondere Umgebungsluft, Pressluft oder ein Inertgas sein kann, wird vor Einströmen in die Probenkammer 4 über einen Gasfilter 16 von störenden Substanzen befreit. Der Gasfilter 16 soll das Trägergas insbesondere von den Substanzen, welche nachgewiesen werden sollen, sowie von Wasserdampf befreien. Eingesetzt werden können insbesondere Absorptionsfilter oder Molekularsiebe. Der Trägergaseinlass 13 kann mittels eines Trägergasventils 17 geschlossen werden. Der Trägergasauslass 14 mündet in eine Zufuhrleitung 18, welche den Gasraum 19 der Probenkammer 4 mit der Sensorkammer 5 verbindet. In der Zufuhrleitung 18 ist ein Trägergasventil 20 vorgesehen, durch das die Zufuhrleitung 18 geöffnet und verschlossen werden kann. Weiterhin weist die Zufuhrleitung 18 einen Trägergasfilter 21 auf, durch den nicht gasförmige Verunreinigungen, nicht jedoch die charakteristischen gas- und dampfförmigen Substanzen aus der Schmierstoffprobe 6 abgeschieden werden.

Durch das wenigstens eine Trägergasventil 20 in der Zufuhrleitung 18 kann die Zufuhr von Trägergas aus der Probenkammer 4 in die Sensorkammer 5 ausgelöst und/oder unterbunden werden.

Die Sensorkammer 5 weist einen Sensorkammereinlass 22 und einen Sensorkammerauslass 23 für Trägergas auf. Der Sensorkammerauslass 23 ist mit dem Gasauslass 24 der-erfindungsgemäßen Vorrichtung verbunden. Dabei kann der Sensorkammerauslass 23 durch das Auslassventil 25 geöffnet oder geschlossen werden.

In der Sensorkammer 5 sind ein oder mehrere Gassensoren 26 angeordnet. In den Figuren sind ein erster Gassensor 26 dargestellt, sowie ein zweiter Gassensor gestrichelt angedeutet. Als geeignete Gassensoren 26 können alle Sensoren eingesetzt werden, die charakteristische gas- bzw. dampfförmige Substanzen, die über den Zustand des zu untersuchenden Stoffes Aufschluss geben, zumindest semi-quantitativ nachweisen. Charakteristische Substanzen sind beispielsweise Oxidationsprodukte (z.B. organische Säuren, Aldehyde, Ketone), Additive (z.B. Antioxidantien, Korrosions- und Verschleißschutzadditive etc.) und deren Zersetzungsprodukte, und Verunreinigungen (wie beispielsweise Wasser, Treibstoffe, Lösemittel). Mehrere Sensoren, die jeweils selektiv oder semi-selektiv verschiedene relevante flüchtige Substanzen aus dem zu untersuchenden Stoff nachweisen, können vorteilhaft kombiniert werden, um die Aussagekraft des Verfahrens zu erhöhen. Verfahrensgemäß kann auch der selektive Nachweis charakteristischer Substanzgruppen statt Einzelsubstanzen zur Beschreibung des Ölzustands herangezogen werden. Beispielsweise kann ein Verhältnis polarer zu unpolarer Substanzen ermittelt und als Maß für die oxidative Belastung eines Stoffes dienen. Erfindungsgemäß können Transducer (z.B. Quarzwaagen SAW- oder IDE-Bauteile) mit chemosensitiven Rezeptorschichten, Halbleiter- oder Metalloxidgasdetektoren, Infrarot oder sonstige optische Gassensoren, Feuchtesensoren oder auch Kombinationen derselben verwendet werden. Vorteilhaft ist auch die Kombination meherer Sensoren auf einem Chip.

Bei Verwendung mehrerer Sensoren wird die Auswertung und Interpretation der multiplen Sensorsignale vorteilhafterweise mit Mustererkennungsalgorithmen, multivarianter Datenanalyse, neuronalen Netzen etc. durchgeführt. Vorteilhafterweise wird dabei mittels einer Trendanalyse die relative und/oder absolute Konzentrationsveränderung von charakteristischen Substanzen bzw. Substanzgruppen als Schmierstoffzustandskriterium ausgewertet. Das kann direkt an der Anlage mit einem integriertem Mikroprozessor oder per Datenfernübertragung an einer spezialisierten Stelle durchgeführt werden.

Besonders aussagekräftige Ergebnisse ergeben sich dann, wenn durch einen ersten Gassensor polare Bestandteile in dem Trägergas und durch einen weiteren Gassensor nicht oder weniger alterungsspezifische Substanzen und/oder Substanzgruppen gemessen werden. Als nicht alterungsspezifische Substanzen kommen z.B. unpolare flüchtige Substanzen in Betracht, deren Konzentration in dem Trägergas sich bei der Alterung des Schmierstoffs nicht oder nur wenig ändert. Indem die Signale dieser Gassensoren ins Verhältnis gesetzt werden, kann eine Aussage über den Alterungszustand des Schmierstoffes erhalten werden. Hierbei kann auch die Entwicklung bzw. der Trend des Verhältnisses über der Zeit berücksichtigt werden.

Weiterhin weist die erfindungsgemäße Vorrichtung Mittel zur Einstellung der physikalischen Bedingungen in der Probenkammer 4 und/oder der Sensorkammer 5 auf. Hierzu ist eine Probenkammerheizung 27 sowie eine Sensorkammerheizung 28 vorgesehen. Die Zufuhrleitung 18 kann durch einen Zufuhrleitungsheizung 29 auf die gewünschte Temperatur gebracht werden. Bei guter Isolierung und kurzen Wegen kann die Zufuhrleitungsheizung 29 ggf. entfallen. Die Heizungen 27 bis 29 werden über Thermostaten gesteuert. Hierzu sind in der Probenkammer 4 und in der Sensorkammer 5 Temperatursensoren 30, 31 und 32 vorgesehen, deren Anschlüsse gestrichelt dargestellt sind. Der Temperatursensor 30 ist im Gasraum 19 angeordnet, während der Temperatursensor 31 die Temperatur des Schmierstoffes in der Probenkammer 4 misst. Der Temperatursensor 32 überwacht die Temperatur der Sensorkammer 5.

Weiterhin weist die erfindungsgemäße Vorrichtung eine Einrichtung zum Spülen der Sensorkammer 5 mittels Trägergas auf, welche unten näher erläutert wird.

Die erfindungsgemäße Vorrichtung weist darüberhinaus eine nicht dargestellte Steuerung für die Betätigung der Trägergasventile 17, 20 und 25 sowie der Ventile 11 und 12 auf.

Für eine Messung wird wie folgt vorgegangen. Zunächst wird bei geöffnetem Ventil 11 Schmierstoff über die Ölzuleitung 7 in die Probenkammer 4 eingebracht. Dabei kann über die Ventile 11 und 12 die Schmierstoffmenge genau eingestellt werden. Zudem wird über den Trägergaseinlass 13 Trägergas in den Gasraum 19 über der Schmierstoffprobe 6 eingebracht. Die Probenkammerheizung 27, welche mittels des Temperatursensors 30 für die Schmierstoffprobe 6 und des Temperatursensors 31 für das Trägergas geregelt wird, erwärmt nun Schmierstoffprobe 6 und das Trägergas auf die gewünschten Temperaturen. Aufgrund des unter Drucks in die gasdichte Probenkammer 4 eingebrachten Trägergases, ist der Druck in der Probenkammer 4 auf den gewünschten Wert eingestellt.

Schmierstoffprobe 6 und Trägergas verbleiben nun in der Probenkammer bis sich das Adsorptions-/Desorptionsgleichgewicht im wesentlichen eingestellt hat. Hierbei reichert sich das Trägergas mit flüchtigen Bestandteilen der Schmierstoffprobe 6 an. Bei diesem Verfahrensschritt sind die Schmierstoffventile 11, 12 sowie die Trägergasventile 17, 20 geschlossen. Nach Ende der Verweilzeit in der Probenkammer 4 wird das Trägergasventil 20 in der Zufuhrleitung zur Sensorkammer 5 geöffnet. Hierdurch kann sich der Überdruck in der Probenkammer 5 entspannen und fördert dadurch angereichertes Trägergas aus der Probenkammer 4 in die Sensorkammer 5.

Anschließend wird in der Sensorkammer 5 wenigstens eine in dem angereicherten Trägergas enthaltene charakteristische flüchtige Substanz und/oder Substanzgruppe aus der Schmierstoffprobe 6 mit dem wenigstens einen Sensor 26 erfasst. Hierbei wird insbesondere die Konzentration der charakteristischen flüchtigen Substanzen und/oder Substanzgruppen durch die Sensoren 26 ermittelt. Die Sensorsignale, welche insbesondere relative und/oder absolute Konzentrationsveränderungen der charakteristischen Substanzen wiedergeben, können durch eine nicht dargestellte Auswerteeinheit ausgewertet und angezeigt werden.

Nach Durchführung der Messung wird das Ventil 12 am Auslass 9 der Probenkammer geöffnet und die Schmierstoffprobe 6 möglichst vollständig abgelassen und beispielsweise wieder dem Schmierstoffreservoir 2 zugeführt.. Dies kann aufgrund der Schwerkraft oder durch den Überdruck des bei geöffnetem Trägergasventil 20 nachströmenden Trägergases erfolgen.

Hierauf kann nach jeder oder nach einer gewissen Anzahl von Messungen eine Spülung der Gassensoren 26 in der Sensorkammer 5 mittels Trägergas erfolgen. Dies geschieht dadurch, dass nach Schließen der Ventils 11, 12 die Trägergasventile 17, 20 sowie das Auslassventil 25 geöffnet und Trägergas durch die geleerte Probenkammer 4 hindurch in die Sensorkammer 5 und weiter zum Gasauslass 24 geleitet wird.

Eine Nullpunktsmessung der gespülten Sensoren kann sich anschließen, um eine Drift durch irreversibel absorbierte Substanzen oder eine anderweitige Schädigung bzw. Verschmutzung der Sensoren zu kompensieren. Anschließend kann der Messzyklus sofort oder gezielt nach einem voreingestellten oder dynamisch an die Betriebsbedingungen der Anlage angepassten Zeitintervall neu beginnen.

Erfindungsgemäß ist es auch möglich, mehrere Schmierstoffe einer Anlage mittels jeweils einer gemeinsamen Sensorkammer 5 zu analysieren. Dementsprechend müssen nur die jeweils erforderlichen Trägergasleitungen und entsprechenden Trägergasventile vorgesehen und so angeordnet werden, dass jeweils das Trägergas aus einer Probenkammer 4 in die Sensorkammer 5 gefördert wird. In gleicher Weise könnte auch eine gemeinsame Probenkammer 4 vorgesehen werden, welche über entsprechende Schmierstoffzuleitungen und Ableitungen mit jeweils einem der Schmierstoffe gefüllt wird.

Die erfindungsgemäße Vorrichtung bzw. das erfindungsgemäße Verfahren können vorteilhaft mit anderen Methoden der Onboard-Überwachung kombiniert werden, z.B. der Viskosimetrie, Dekametrie, Partikelzählung und dergleichen. Damit kann die Aussagekraft der Überwachung noch gesteigert werden. Die entsprechenden Sensoren lassen sich ggf. in die Probenkammer integrieren. Dadurch profitieren auch diese ergänzenden Sensoren von den klar definierten Bedingungen in der Probenkammer: Feste Temperatur, beruhigte Zone etc.. Die Signale dieser zusätzlichen Sensoren können ebenfalls in die Auswertealgorithmen einbezogen werden. Vorteil der vorliegenden Erfindung ist, dass die die Messergebnisse beeinflussenden Größen sorgfältig minimiert werden können. Dies beruht insbesondere darauf, dass in der Probenkammer ein definiertes Schmierstoffproben- und Gasvolumen, definierte Temperaturen und Temperaturgradienten sowie ein gleichbleibendes Trägergas vorgegeben werden. Damit entfällt die Abhängigkeit der Messergebnisse von den Anlagenbedingungen wie Betriebs- und Außentemperatur, Füllstand in der Anlage, Feuchte und Ölgehalt der Umgebungsluft. Daher kann erfindungsgemäß auch die Notwendigkeit einer zeit und kostenaufwendigen Bestimmung der anlagespezifischen Korrekturfunktionen zur Signalauswertung entfallen.

Die erfindungsgemäße Gestaltung macht es dabei nicht nur möglich, die Präzision der Messungen zu erhöhen. Darüber hinaus kann die Lebensdauer der Anlage, insbesondere der eingesetzten Sensoren erheblich verlängert werden, da aufgrund der Erfindung eine diskontinuierliche, insbesondere in regelmäßigen Abständen wiederkehrende Messung der flüchtigen Bestandteile erfolgen kann. Dementsprechend sind die Sensoren nur zeitweise den in dem angereicherten Trägergas enthaltenen aggressiven oder verschmutzenden Substanzen ausgesetzt.

Darüber hinaus ergibt sich erfindungsgemäß durch den Einbau der Sensoren in eine spezielle Sensorkammer mit Trägergaszufuhr eine erhebliche Steigerung der Sensorlebensdauer. Einerseits können durch Spülung mit Trägergas, ggf. bei erhöhter Sensortemperatur, Verunreinigungen auf den Sensoren wieder abgetragen werden. Andererseits sind die Sensoren nur während der aktuellen Messung den potentiell aggressiven flüchtigen Substanzen aus dem zu überwachenden Stoff ausgesetzt, sonst befinden sie sich im neutralen Trägergas. Eine intermittierende, den Betriebsbedingungen und dem Überwachungsaufwand angepasste Messhäufigkeit kann damit die tatsächliche Belastung des Überwachungssystems erheblich verringern. Auch eine Überhitzung der Sensoren durch ungewöhnliche Betriebsbedingungen der zu überwachenden Anlage kann durch die vorteilhafte räumliche Trennung von Anlage und Sensorkammer gewährleistet werden.

### Bezugszeichenliste:

- 1: Anlage
- 2: Ölreservoir
- 3: Ölumlaufschmierung
- 4: Probenkammer
- 5: Sensorkammer
- 6: Schmierstoffprobe
- 7: Ölzuleitung
- 8: Einlaß
- 9: Auslaß
- 10: Ölablaufleitung
- 11: Ventil (Einlaß)
- 12: Ventil (Auslaß)
- 13: Trägergaseinlaß
- 14: Trägergasauslaß
- 15: Gasversorgung
- 16: Gasfilter
- 17: Trägergasventil
- 18: Zufuhrleitung
- 19: Gasraum
- 20: Trägergasventil
- 21: Trägergasfilter
- 22: Sensorkammereinlaß
- 23: Sensorkammerauslaß
- 24: Gasauslaß
- 25: Trägergasventil
- 26: Gassensor(en)
- 27: Probenkammerheizung
- 28: Sensorkammerheizung
- 29: Zufuhrleitungsheizung
- 30: Temperatursensor (Gasraum)
- 31: Temperatursensor (Öl)
- 32: Temperatursensor (Sensorkammer)

## Patentansprüche

1. Vorrichtung zum automatischen Überwachen des Gebrauchszustandes eines Schmierstoffes einer Maschine oder eines Maschinenteils, mit wenigstens einem Gassensor (26) und mit einer Probenkammer (4), welche einen Raum zur Aufnahme einer Schmierstoffprobe (6), wenigstens einen Einlass (13) und Auslass (14) für Trägergas und einen Gasraum (19) über der Schmierstoffprobe (6), in dem sich das Trägergas mit flüchtigen Bestandteilen des Schmierstoffes anreichern kann, aufweist, wobei Mittel zur Zufuhr einer Schmierstoffprobe aus dem Schmiersystem der Maschine oder des Maschinenteils in die Probenkammer (4) **dadurch gekennzeichnet, daß** Mittel (27) zur Einstellung der physikalischen Bedingungen in der Probenkammer (4), welche eine Heizung (27) oder Kühlung aufweisen, vorgesehen sind und wobei der Gassensor (26) ein von der Konzentration eines oder mehrerer flüchtiger Bestandteile in dem angereicherten Trägergas abhängiges Signal erzeugt.

2. Vorrichtung nach Anspruch 1, **dadurch gekennzeichnet, dass** der wenigstens eine Gassensor (26) in einer Sensorkammer (5) angeordnet ist und dass der Gasraum (19) der Probenkammer (4) mit der Sensorkammer (5) durch eine Zufuhrleitung (18) verbunden ist.

3. Vorrichtung nach Anspruch 2, **dadurch gekennzeichnet, dass** Mittel (28) zur Einstellung der physikalischen Bedingungen in der Sensorkammer (5) vorgesehen sind.

4. Vorrichtung nach Anspruch 2 oder 3, **gekennzeichnet durch** wenigstens ein Trägergasventil (20) in der Zufuhrleitung (18) zwischen der Probenkammer (4) und der Sensorkammer (5).

5. Vorrichtung nach Anspruch 4, **dadurch gekennzeichnet, dass** durch das wenigstens eine Trägergasventil (20) in der Zufuhrleitung (18) zwischen Probenkammer (4) und Sensorkammer (5) die Zufuhr von Trägergas aus Probenkammer (4) in die Sensorkammer (5) ausgelöst und/oder unterbunden werden kann.

6. Vorrichtung nach einem der Ansprüche 2 bis 5, **dadurch gekennzeichnet, dass** für die Sensorkammer (5) eine über einen Thermostat gesteuerte Heizung (28) oder Kühlung vorgesehen ist.

7. Vorrichtung nach einem der vorangehenden Ansprüche, **dadurch gekennzeichnet, dass** die Mittel zur Zufuhr einer Schmierstoffprobe aus dem Schmiersystem der Maschine oder des Maschinenteils den Schmierstoff aus einem Schmierstoflkreislauf (3) der Maschine oder des Maschinenteils in die Probenkammer (4) fördem.

8. Vorrichtung nach einem der vorangehenden Ansprüche, **dadurch gekennzeichnet, dass** die Probenkammer (4) wenigstens einen Einlass (8) und wenigstens einen Auslass (9) für Schmierstoff aufweist.

9. Vorrichtung nach Anspruch 8, **dadurch gekennzeichnet, dass** der Auslass (9) derart ausgebildet ist, dass im wesentlichen eine vollständige Entleerung der Probenkammer (4) erfolgen kann.

10. Vorrichtung nach einem der vorangehenden Ansprüche, **dadurch gekennzeichnet, dass** Mittel zum Einbringen von Trägergas in die Probenkammer (4), insbesondere unter Überdruck, vorgesehen sind.

11. Vorrichtung nach einem der vorangehenden Ansprüche, **dadurch gekennzeichnet, dass** eine Einrichtung zum Spülen der Probenkammer (4) und/oder der Sensorkammer (5) mittels Trägergas vorgesehen ist.

12. Vorrichtung nach Anspruch 11, **dadurch gekennzeichnet, dass** zum Spülen eine Spülleitung (51) vorgesehen ist, welche die Zufuhr von sauberem Trägergas in die Sensorkammer (5) ermöglicht.

13. Vorrichtung nach einem der vorangehenden Ansprüche, **dadurch gekennzeichnet, dass** eine Steuerung für das Trägergasventil (20) vorgesehen ist, welche intermittierend die Füllung der Sensorkammer (4) mit angereichertem Trägergas ermöglicht.

14. Verfahren zur automatischen Überwachung des Gebrauchszustandes eines Schmierstoffes einer Maschine oder eines Maschinenteils mit folgenden Schritten:
- Einbringen einer Schmierstoffprobe aus der Maschine oder dem Maschinenteil in eine Probenkammer (4) durch Mittel zur Zufuhr einer Schmierstoffprobe aus dem Schmiersystem der Maschine oder des Maschinenteils in die Probenkammer und Einbringen von Trägergas in die Probenkammer (4),
**dadurch gekennzeichnet daß**
- Einstellen der physikalischen Probenbedingungen, nämlich der Temperatur in der Probenkammer (4) durch eine Heizung oder Kühlung auf einen vorbestimmten Wert,
- Anreicherung des Trägergases mit flüchtigen Bestandteilen der Schmierstoffprobe (6),
- Erfassung von wenigstens einer in dem angereicherten Trägergas enthaltenen charakteristischen flüchtigen Substanz und/oder Substanzgruppe aus der Schmierstoffprobe mit wenigstens einem Gassensor (26).

15. Verfahren nach Anspruch 14, **dadurch gekennzeichnet, dass** das angereicherte Trägergas nach der Anreicherung mit flüchtigen Substanzen und/oder Substanzgruppen über eine Zufuhrleitung (18) in eine Sensorkammer (5) geleitet wird, in der die Erfassung von wenigstens einer dem angereicherten Trägergas enthaltenen charakteristischen flüchtigen Substanz und/oder Substanzgruppe aus der Schmierstoffprobe mit dem wenigstens einen Gassensor (26) erfolgt.

16. Verfahren nach Anspruch 15, **dadurch gekennzeichnet, dass** die Zufuhr des angereicherten Trägergases zur Sensorkammer (5) durch Öffnen eines in der Zufuhrleitung (18) angeordneten Trägergasventils (20) ausgelöst und durch Schließen des Trägergasventils (20) unterbrochen wird.

17. Verfahren nach einem der Ansprüche 14 bis 16, **dadurch gekennzeichnet, dass** das Trägergas in die Probenkammer (4) unter Überdruck eingebracht wird.

18. Verfahren nach Anspruch 16 und 17, **dadurch gekennzeichnet, dass** das angereicherte Trägergas aus der Probenkammer (4) aufgrund Entspannens des Überdrucks in der Probenkammer (4) durch Öffnen des in der Zufuhrleitung (18) zur Sensorkammer angeordneten Trägergasventils (17) in die Sensorkammer (5) transportiert wird.

19. Verfahren nach Anspruch einem der Ansprüche 14 bis 18, **dadurch gekennzeichnet, dass** für aufeinanderfolgende Messungen stets die gleiche Menge Schmierstoffprobe (6) und/oder Trägergas in die Probenkammer (4) eingebracht wird.

20. Verfahren nach einem der Ansprüche 14 bis 19, **dadurch gekennzeichnet, dass** die Probenkammer (4) und/oder die Sensorkammer (5) nach einer oder mehreren Messungen mit sauberem Trägergas gespült wird.

21. Verfahren nach einem der Ansprüche 14 bis 20, **dadurch gekennzeichnet, dass** zur Spülung die in der Probenkammer (4) enthaltene Schmierstoffprobe (6) entfernt und dann Trägergas durch die Probenkammer (4) und nachfolgend durch die Sensorkammer (5) geleitet wird.

22. Verfahren nach einem der Ansprüche 14 bis 21, **dadurch gekennzeichnet, dass** die Spülung durch eine in die Sensorkammer (5) mündende Spülleitung (51) erfolgt.

23. Verfahren nach einem der Ansprüche 14 bis 22, **dadurch gekennzeichnet, dass** das Trägergas vor dem Einbringen in die Probenkammer (4) gereinigt, insbesondere gefiltert wird.

24. Verfahren nach einem der Ansprüche 14 bis 23, **dadurch gekennzeichnet, dass** als Trägergas Umgebungsluft, Pressluft oder Inertgas verwendet wird.

25. Verfahren nach einem der Ansprüche 14 bis 24, **dadurch gekennzeichnet, dass** durch wenigstens einen der Gassensoren polare charakteristischen flüchtige Substanzen und/oder Substanzgruppen oder deren Konzentration und durch wenigstens einen weiteren Gassensor nicht oder weniger alterungsspezifische charakteristische flüchtige Substanzen und/oder Substanzgruppen, insbesondere unpolare flüchtige Substanzen und/oder Substanzgruppen, oder deren Konzentration detektiert werden.

26. Verfahren nach Anspruch 25, **dadurch gekennzeichnet, dass** das Verhältnis von polaren zu nicht oder wenig alterungsspezifischen flüchtigen Substanzen und/oder Substanzgruppen ausgewertet und als Maß für den Gebrauchszustand des Öls verwendet wird.

## Claims

1. Device for automatically monitoring the condition of use of a lubricant of a machine or machine part, having at least one gas sensor (26) and having a sample chamber (4) which has a space for holding a lubricant sample (6), at least one inlet (13) and outlet (14) for carrier gas, and a gas space (19) above the lubricant sample (6) in which the carrier gas can be enriched with volatile components of the lubricant, there being provided means for feeding a lubricant sample from the lubricant system of the machine or machine part into the sample chamber (4) and means (27) for setting the physical conditions in the sample chamber (4), which means have a heater (27) or cooler, and the gas sensor (26) generating a signal dependent on the concentration of one or more volatile components in the enriched carrier gas.

2. Device according to Claim 1, **characterized in that** the at least one gas sensor (26) is arranged in a sensor chamber (5), and **in that** the gas space (19) of the sample chamber (4) is connected to the sensor chamber (5) by a feed line (18).

3. Device according to Claim 2, **characterized in that** means (28) are provided for setting the physical conditions in the sensor chamber (5).

4. Device according to Claim 2 or 3, **characterized by** at least one carrier gas valve (20) in the feed line (18) between the sample chamber (4) and the sensor chamber (5).

5. Device according to Claim 4, **characterized in that** the feeding of carrier gas from the sample chamber (4) into the sensor chamber (5) can be initiated and/or suppressed by the at least one carrier gas valve (20) in the feed line (18) between the sample chamber (4) and sensor chamber (5).

6. Device according to one of Claims 2 to 5, **characterized in that** a thermostatically controlled heater (28) or cooler is provided for the sensor chamber (5).

7. Device according to one of the preceding claims, **characterized in that** the means for feeding a lubricant probe from the lubricant system of the machine or machine part convey the lubricant from a lubricant circuit (3) of the machine or machine part into the sample chamber (4).

8. Device according to one of the preceding claims, **characterized in that** the sample chamber (4) has at least one inlet (8) and at least one outlet (9) for lubricant.

9. Device according to Claim 8, **characterized in that** the outlet (9) is designed in such a way that the sample chamber (4) can be substantially completely emptied.

10. Device according to one of the preceding claims, **characterized in that** means are provided for introducing carrier gas into the sample chamber (4), in particular under excess pressure.

11. Device according to one of the preceding claims, **characterized in that** a device is provided for flushing the sample chamber (4) and/or the sensor chamber (5) by means of carrier gas.

12. Device according to Claim 11, **characterized in that** provided for flushing is a flushing line (51) which enables the feeding of clean carrier gas in to the sensor chamber (5).

13. Device according to one of the preceding claims, **characterized in that** provided for the carrier gas valve (20) is a controller which enables the sensor chamber (4) to be intermittently filled with enriched carrier gas.

14. Method for automatically monitoring the condition of use of a lubricant of a machine or machine part, having the following steps:
- introducing a lubricant sample from the machine or machine part into a sample chamber (4) by using means for feeding a lubricant sample from the lubricant system of the machine or machine part into the sample chamber, and introducing carrier gas into the sample chamber (4),
- setting the physical sample conditions, specifically the temperature in the sample chamber (4) to a prescribed value by means of heater or cooler,
- enriching the carrier gas with volatile components of the lubricant sample (6),
- determining at least one characteristic volatile substance and/or substance group, contained in the enriched carrier gas, from the lubricant sample with the aid of at least one gas sensor (26).

15. Method according to Claim 14, **characterized in that** after being enriched with volatile substances and/or substance groups the enriched carrier gas is led via a feed line (18) into a sensor chamber (5) in which at least one characteristic volatile substance and/or substance group, contained in the enriched carrier gas, from the lubricant sample is detected with the aid of the at least one gas sensor (26).

16. Method according to Claim 15, **characterized in that** the feeding of the enriched carrier gas to the sensor chamber (5) is initiated by opening a carrier gas valve (20) arranged in the feed line (18), and is interrupted by closing the carrier gas valve (20).

17. Method according to one of Claims 14 to 16, **characterized in that** the carrier gas is introduced into the sample chamber (4) under excess pressure.

18. Method according to Claims 16 and 17, **characterized in that** the enriched carrier gas is transported into the sensor chamber (5) from the sample chamber (4) by relieving the excess pressure in the sample chamber (4) by opening the carrier gas valve (17) arranged in the feed line (18) to the sensor chamber.

19. Method according to one of Claims 14 to 18, **characterized in that** always the same quantity of lubricant sample (6) and/or carrier gas is introduced into the sample chamber (4) for the purpose of consecutive measurements.

20. Method according to one of Claims 14 to 19, **characterized in that** the sample chamber (4) and/or the sensor chamber (5) are/is flushed with clean carrier gas after one or more measurements.

21. Method according to one of Claims 14 to 20, **characterized in that** for flushing purposes the lubricant sample (6) contained in the sample chamber (4) is removed and then carrier gas is led through the sample chamber (4) and subsequently through the sensor chamber (5).

22. Method according to one of Claims 14 to 21, **characterized in that** the flushing is performed by a flushing line (51) which opens into the sensor chamber (5).

23. Method according to one of Claims 14 to 22, **characterized in that** the carrier gas is cleaned, in particular filtered, before being introduced into the sample chamber (4).

24. Method according to one of Claims 14 to 23, **characterized in that** ambient air, compressed air or inert gas is used as carrier gas.

25. Method according to one of Claims 14 to 24, **characterized in that** at least one of the gas sensors detects polar characteristic volatile substances and/or substance groups or the concentration thereof, and at least one further sensor detects characteristic volatile substances and/or substance groups which are not, or are less, aging-specific, in particular nonpolar volatile substances and/or substance groups or the concentration thereof.

26. Method according to Claim 25, **characterized in that** the ratio of polar volatile substances and/or substance groups to volatile substances and/or substance groups which are not, or are scarcely, aging-specific is evaluated and used as a measure of the condition of use of the oil.

## Revendications

1. Dispositif de surveillance automatique de l'état d'usure du lubrifiant d'une machine ou d'une pièce de machine, qui présente au moins un détecteur de gaz (26) et une chambre (4) à échantillon qui présente un espace de reprise d'un échantillon de lubrifiant (6), au moins un orifice d'admission (13) et un orifice de sortie (14) pour le gaz porteur et une chambre à gaz (19) située au-dessus de l'échantillon de lubrifiant (6) et dans laquelle le gaz porteur peut s'enrichir en composants volatils du lubrifiant, des moyens d'amenée d'un échantillon de lubrifiant du système de lubrification de la machine ou de la pièce de machine dans la chambre (4) à échantillon et des moyens (27) d'ajustement des conditions physiques dans la chambre (4) à échantillon qui présentent un chauffage (27) ou un refroidissement étant prévus, le détecteur de gaz (26) formant un signal qui dépend de la concentration d'un ou plusieurs composants volatils présents dans le gaz porteur enrichi.

2. Dispositif selon la revendication 1, **caractérisé en ce que** le ou les détecteurs de gaz (26) sont disposés dans une chambre (5) à détecteurs et **en ce que** la chambre à gaz (19) de la chambre (4) à échantillon est reliée à la chambre (5) à détecteurs par un conduit d'amenée (18).

3. Dispositif selon la revendication 2, **caractérisé en ce qu'**il présente des moyens (28) d'ajustement des conditions physiques dans la chambre (5) à détecteurs.

4. Dispositif selon les revendications 2 ou 3, **caractérisé par** au moins une soupape (20) de gaz porteur disposée dans le conduit d'amenée (18) entre la chambre (4) à échantillon et la chambre (5) à détecteurs.

5. Dispositif selon la revendication 4, **caractérisé en ce que** l'apport de gaz porteur provenant de la chambre (4) à échantillon jusque dans la chambre (5) à détecteurs peut être déclenché et/ ou interrompu par la ou les soupapes (20) à gaz porteur prévues dans le conduit d'amenée (18) entre la chambre (4) à échantillon et la chambre (5) à détecteurs.

6. Dispositif selon l'une des revendications 2 à 5, **caractérisé en ce qu'**un chauffage (28) ou un refroidissement commandés par un thermostat sont prévus pour la chambre (5) à détecteurs.

7. Dispositif selon l'une des revendications précédentes, **caractérisé en ce que** les moyens d'amenée d'un échantillon de lubrifiant du système de lubrification de la machine ou de la pièce de machine transportent dans la chambre (4) à échantillon le lubrifiant qui provient d'un circuit (3) de lubrifiant de la machine ou de la pièce de machine.

8. Dispositif selon l'une des revendications précédentes, **caractérisé en ce que** la chambre (4) à échantillon présente au moins un orifice d'admission (8) et au moins un orifice de sortie (9) de lubrifiant.

9. Dispositif selon la revendication 8, **caractérisé en ce que** l'orifice de sortie (9) est configuré de manière à permettre de vider essentiellement complètement la chambre (4) à échantillon.

10. Dispositif selon l'une des revendications précédentes, **caractérisé en ce qu'**il présente des moyens d'amenée du gaz porteur dans la chambre (4) à échantillon, en particulier sous dépression.

11. Dispositif selon l'une des revendications précédentes, **caractérisé en ce qu'**il présente un dispositif de rinçage de la chambre (4) à échantillon et/ ou de la chambre (5) à détecteurs au moyen du gaz porteur.

12. Dispositif selon la revendication 11, **caractérisé en ce que** pour le rinçage, il présente un conduit de rinçage (51) qui permet l'amenée de gaz porteur propre dans la chambre (5) à détecteurs.

13. Dispositif selon l'une des revendications précédentes, **caractérisé en ce qu'**il présente une commande de la soupape (20) de gaz porteur qui permet de remplir par intermittence la chambre à détecteurs (4) en gaz porteur enrichi.

14. Procédé de surveillance automatique de l'état d'usure d'un lubrifiant d'une machine ou d'une pièce de machine, lequel procédé comprend les étapes qui consistent à :
- amener un échantillon de lubrifiant de la machine ou de la pièce de machine dans une chambre (4) à échantillon par des moyens d'amenée d'un échantillon de lubrifiant du système de lubrification de la machine ou de la pièce de machine dans la chambre à échantillons et amener du gaz porteur dans la chambre (4) à échantillon,
- par un chauffage ou un refroidissement, régler à une valeur prédéterminée les conditions physiques de l'échantillon, à savoir sa température, dans la chambre (4) à échantillon,
- enrichir le gaz porteur en composants volatils de l'échantillon de lubrifiant (6) et
- à l'aide d'au moins un détecteur de gaz (26), détecter au moins une substance volatile et/ou un groupe de substances volatiles caractéristiques de l'échantillon de lubrifiant et que contient le gaz porteur enrichi.

15. Procédé selon la revendication 14, **caractérisé en ce qu'**après avoir été enrichi en substances et/ou groupes de substances volatiles, le gaz porteur enrichi est conduit par un conduit d'amenée (18) dans une chambre (5) à détecteurs dans laquelle la détection d'au moins une substance volatile et/ ou d'un groupe de substances volatiles caractéristiques de l'échantillon de lubrifiant que contient le gaz porteur enrichi s'effectue à l'aide du ou des détecteurs de gaz (26).

16. Procédé selon la revendication 15, **caractérisé en ce que** l'amenée du gaz porteur enrichi dans la chambre (5) à détecteurs est déclenchée par ouverture d'une soupape (20) de gaz porteur disposée dans le conduit d'amenée (18) et est interrompue par fermeture de la soupape (20) de gaz porteur.

17. Procédé selon l'une des revendications 14 à 16, **caractérisé en ce que** le gaz porteur est apporté sous dépression dans la chambre (4) à échantillon.

18. Procédé selon les revendications 16 et 17, **caractérisé en ce que** le gaz porteur enrichi qui provient de la chambre (4) à échantillon est transporté dans la chambre (5) à détecteurs par détente de la dépression qui règne dans la chambre (4) à échantillon par ouverture de la soupape (17) de gaz porteur disposée dans le conduit d'amenée (18) qui débouche dans la chambre à détecteurs.

19. Procédé selon l'une des revendications 14 à 18, **caractérisé en ce que** la quantité d'échantillons de lubrifiant (6) et/ou de gaz porteur amenée dans la chambre (4) à échantillon est toujours la même dans des mesures successives.

20. Procédé selon l'une des revendications 14 à 19, **caractérisé en ce qu'**après une ou plusieurs mesures, la chambre (4) à échantillon et/ ou la chambre (5) à détecteurs sont rincées par du gaz porteur propre.

21. Procédé selon l'une des revendications 14 à 20, **caractérisé en ce que** pour le rinçage, l'échantillon de lubrifiant (6) est retiré de la chambre (4) à échantillon et **en ce que** le gaz porteur est conduit à travers la chambre (4) à échantillon et ensuite à travers la chambre (5) à détecteurs.

22. Procédé selon l'une des revendications 14 à 21, **caractérisé en ce que** le balayage s'effectue grâce à un conduit de balayage (51) qui débouche dans la chambre (5) à détecteurs.

23. Procédé selon l'une des revendications 14 à 22, **caractérisé en ce que** le gaz porteur est nettoyé et en particulier filtré avant d'être introduit dans la chambre (4) à échantillon.

24. Procédé selon l'une des revendications 14 à 23, **caractérisé en ce que** l'on utilise comme gaz porteur l'air ambiant, de l'air comprimé ou un gaz inerte.

25. Procédé selon l'une des revendications 14 à 24, **caractérisé en ce que** l'on détecte des substances volatiles et/ou des groupes de substances volatiles polaires caractéristiques ou leur concentration par au moins l'un des détecteurs de gaz et **en ce qu'**à l'aide d'au moins un autre détecteur de gaz, on détecte des substances volatiles et/ou des groupes de substances volatiles caractéristiques, en particulier des substances volatiles non polaires et/ou des groupes de substances volatiles non polaires, non spécifiques ou moins spécifiques du vieillissement, ou leur concentration.

26. Procédé selon la revendication 25, **caractérisé en ce qu'**on évalue le rapport entre les substances volatiles polaires et/ou le groupe de substances volatiles polaires et les substances volatiles ou groupes de substances volatiles non spécifiques ou moins spécifiques au vieillissement et qu'on l'utilise comme mesure de l'état d'usure de l'huile.
